# EUROPEAN PATENT SPECIFICATION

(11) **EP 4 173 563 B1**
(45) Date of publication and mention of the grant of the patent: **17.07.2024**
(21) Application number: 22202583.5
(22) Date of filing: 19.10.2022
(51) Int. Cl.: A61B 5/0537, A61B 5/107, G01G 19/50

(54) **BODY COMPOSITION ANALYSIS SYSTEM HAVING IMAGE SCANNING FUNCTION**
SYSTEM ZUR ANALYSE DER KÖRPERZUSAMMENSETZUNG MIT BILDABTASTFUNKTION
SYSTÈME D'ANALYSE DE COMPOSITION CORPORELLE AYANT UNE FONCTION DE BALAYAGE D'IMAGE

(30) Priority: 26.10.2021 TW 110139711
(43) Date of publication of application: 03.05.2023
(73) Proprietor: Starbia Meditek Co., Ltd., Taichung City (TW)
(72) Inventor: HSIEH, Kuen-Chang, Taichung City (TW); TSAI, Chih-Ching, Taichung (TW)
(74) Representative: Straus, Alexander

(56) References cited:
- CN-U- 208 635 907
- JP-B2- 3 692 335
- US-A1- 2006 140 463
- US-A1- 2014 340 479

## Description

### BACKGROUND OF THE INVENTION

### 1. Field of the Invention

The present invention relates to body composition analysis techniques and more particularly, to a body composition analysis system having image scanning function.

### 2. Description of the Related Art

Body composition can be assessed by a plurality of different manners, including advanced medical imaging equipment, such as dual-energy X-ray absorptiometry (DXA), computed tomography (CT) and magnetic resonance imaging (MRI), which can provide assessments relatively higher in accuracy, but the examination costs high and requires specialists for operation. Besides, the radiation dose from the computed tomography may reach hundreds of times that from the ordinary X-ray, so there are limitations in its use. In contrast, the dual-energy X-ray absorptiometry is extremely low in radiation dose and high in accuracy. It has become the gold standard for body composition analysis, but still unsuitable for routine examinations, due to the aforementioned problems of high cost and requiring specialists for operation. There are other manners, such as underwater weighing, air displacement plethysmography (ADP), skinfold measurement, circumference measurement, isotope dilution, ultrasound measurement, potassium-40 counting, and bioelectrical impedance analysis (BIA).

Wherein, the bioelectrical impedance analysis is currently the most commonly used manner for assessing human body composing components, because its operation is fast, simple, relatively safe and non-invasive. For the bioelectrical impedance analysis, the measured person lies or stands on the body composition measuring instrument. The four limbs of the measured person contact electrodes of the body composition measuring instrument, and very low current is passed therethrough. By the characteristic that the difference of the water content and cell membrane properties of different tissues in the body results in different resistance and reactance for current of different frequencies, the body water content is obtained. The traditional manner is usually performed in coordination with information of the measured person, such as age, sex, weight and height. Estimating equations are used to obtain various composing components of the human body, including body fat weight, non-fat weight, total water content, intracellular and extracellular fluid volume, and then further calculate the percentages or content of bones, muscles, fat and other tissues.

In recent years, the application of three-dimensional body scanning technology for human body composition analysis has become more and more common and has become an emerging research direction. For example, in US Patent Publication No. 2019/0223788 A1, for solving the problem that in three-dimensional imaging manner the body fat percentage is calculated by directly using volume and thereby not accurate enough, the primarily raised technique is using three-dimensional body model to determine the body volume and geometric shape to create a human impedance model including multiple segments of cylindrical or conical form, which is the model introducing multiple segments into each segment of the body, especially the shoulder joint, upper arm, fore arm, hand, thigh, knee joint or calf. After that, at least two electrodes are used to measure impedance of the human body to obtain detailed impedance of the body segments especially. At last, by adding the partial body fat content of each segment, the body fat content of the whole body is obtained.

However, for the human body photography provided in the aforementioned patent, the measurement is performed in a manner of standing and both hands contacting the electrodes, which has at least two disadvantages to be improved:
1. When standing on a rotary plate for rotary scanning, the measured person will inevitably swing or change posture, thereby affecting the resistance and reactance measured by the body composition measuring instrument, resulting in that the measurement results are not accurate enough.
2. When the measured person contacts the electrodes by the both hands, there are included angles between the arms and the trunk. If the included angle is too small, the skin of the arm will be in contact with the skin of the trunk, or the clothing will bring contact problem, so that the resistance and reactance measured by the body composition measuring instrument will be affected and thereby having error, resulting in that the measurement is unable to have repeatability and not accurate. If being required to maintain a certain included angle between the arm and the trunk, the measured person is liable to swing or change posture, that also makes the measurement results not accurate enough. US 2014/340479 A1 discloses a health system and in particular to a system and method for capturing and processing body measurements. It discloses a system comprising: one or more body scanners, each body scanner configured to capture a plurality depth images of a user; a backend system, coupled to each body scanner, configured to receive the plurality of depth images, generate a three dimensional avatar of the user based on the plurality of depth images, and generate a set of body measurements of the user based on the avatar; and a repository in communication with the backend system configured to associate the body measurement data with the user and store the body measurement data. The document further discloses telescopic rods for user adjustment. JP 3 692335 B2 discloses a body componential analysis apparatus of handle adjustment which can be freely carried out with respect to a case. The document further discloses pivotable rods for user adjustment. US 2006/140463 A1 discloses a three-dimensional, digitized capturing of the shape bodies and body parts using mechanically positioned imaging sensors. CN 208 635 907 U discloses a three-dimensional human body scanning device with built-in weighing module.

### SUMMARY OF THE INVENTION

It is a primary objective of the present invention to provide a body composition analysis system having image scanning function, which can prevent the measured person from swinging or changing posture, thereby improving the accuracy of the measurement results when compared with the conventional techniques.

It is another objective of the present invention to provide a body composition analysis system having image scanning function, which is adjustable according to the body shape of the measured person to maintain the posture of the measured person at the most appropriate measured condition, and able to provide the measured person support so that the measured person is not liable to swing or change posture, thereby improving the repeatability and accuracy of the measurement results when compared with the conventional techniques.

To attain the above objective, the present invention provides a body composition analysis system having image scanning function, which includes an image capturing device, a body composition analyzer distanced from the image capturing device for a predetermined distance, and a control panel. The body composition analyzer has a stand platform, two handrails and a calculating unit. The stand platform is provided with two first electrode sets. The two handrails each have a telescopic rod and a handle. An end of each of the telescopic rods is pivotably attached to the stand platform, and each of the handles is disposed at another end of each of the telescopic rods. Each of the handles has a second electrode set. The calculating unit is electrically connected with the image capturing device. The calculating unit includes a body shape module database and a calculational logic. The calculating unit receives resistance and reactance measured by the two first electrode sets and the two second electrode sets and receives image data obtained by the image capturing device, and after comparing the image data with the body shape module database, calculates by the calculational logic to obtain a body composition data. The control panel is electrically connected with the body composition analyzer, the image capturing device and the calculating unit for showing the body composition data.

As a result, in the body composition analysis system having image scanning function provided by the present invention, the technical feature of disposing the two handrails on the stand platform can prevent the measured person from swinging or changing posture, thereby improving the accuracy of the measurement results.

Besides, in the body composition analysis system having image scanning function provided by the present invention, the technical feature of disposing the two handrails on the stand platform can provide convenience to the body composition data measurement for the measured person who needs an auxiliary or handrails to stand.

In addition, in the body composition analysis system having image scanning function provided by the present invention, the technical feature of the two telescopic rods and the two telescopic rods being pivotably attached to the stand platform enables the angles of the two handrails adjustment according to the body shape of the measured person to maintain the posture of the measured person at the most appropriate measured condition and provide the measured person support so that the measured person is not liable to swing or change posture, which can make the measurement results have repeatability and improve the accuracy thereof.

### BRIEF DESCRIPTION OF THE DRAWINGS

FIG. 1 is a schematic view of the structure of a first preferred embodiment of the present invention.
FIG. 2 is a block diagram of the first preferred embodiment of the present invention.
FIG. 3 is a schematic view showing the usage condition of the first preferred embodiment of the present invention.
FIG. 4 is a schematic view of the structure of a second preferred embodiment of the present invention.

### DETAILED DESCRIPTION OF THE INVENTION

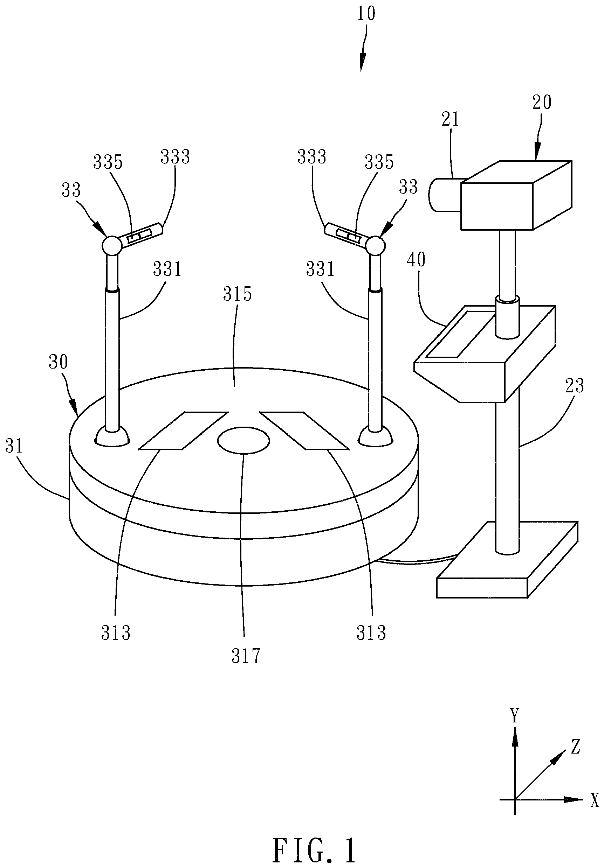

For the detailed description of the technical features of the present invention, a first preferred embodiment is described hereunder in coordination with FIGS. 1-4, wherein a body composition analysis system 10 having image scanning function of the present invention primarily includes an image capturing device 20, a body composition analyzer 30, and a control panel 40.

As shown in FIG. 1, the body composition analyzer 30 is distanced from the image capturing device 20 for a predetermined distance. The predetermined distance is set according to the requirement of the image data to be obtained by the image capturing device 20, such as obtaining the image data of a part or the whole of the body of the measured person.

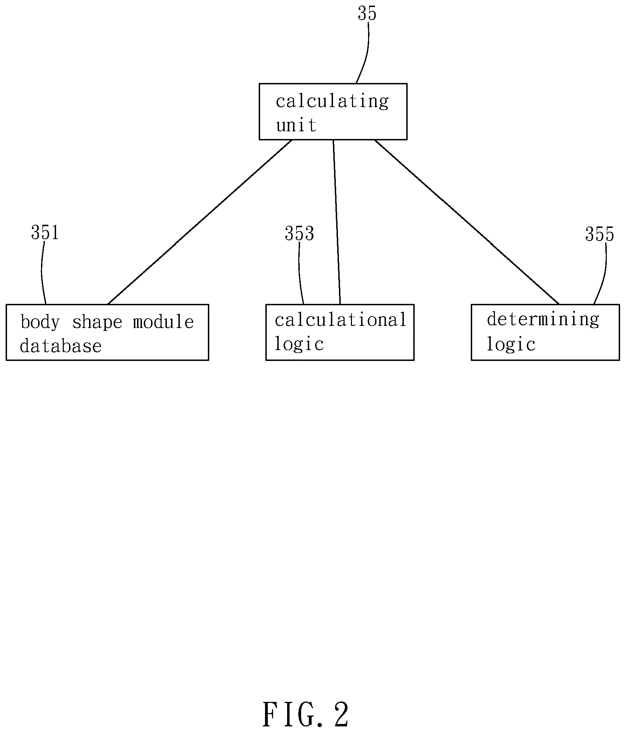

As shown in FIGS. 1 and 2, the body composition analyzer 30 has a stand platform 31, two handrails 33 and a calculating unit 35. The stand platform 31 is provided with two first electrode sets 313. The two handrails 33 each have a telescopic rod 331 and a handle 333. An end of each of the telescopic rods 331 is pivotably attached to the stand platform 31, and each of the handles 333 is disposed at another end of each of the telescopic rods 331. Each of the handles 333 has a second electrode set 335. The calculating unit 35 is electrically connected with the image capturing device 20, the two first electrode sets 313 and the two second electrode sets 335. The calculating unit 35 includes a body shape module database 351 and a calculational logic 353. The calculating unit 35 receives resistance and reactance measured by the two first electrode sets 313 and the two second electrode sets 335 and receives image data obtained by the image capturing device 20, and after comparing the image data obtained by the image capturing device 20 with the body shape module database 351, calculates by the calculational logic 353 to obtain a body composition data.

The control panel 40 is electrically connected with the body composition analyzer 30, the image capturing device 20 and the calculating unit 35 for showing the body composition data.

Therefore, in the body composition analysis system 10 having image scanning function provided by the present invention, the technical feature of disposing the two handrails 33 on the stand platform 31 can prevent the measured person from swinging or changing posture, thereby improving the accuracy of the measurement results.

Besides, in the body composition analysis system 10 having image scanning function provided by the present invention, the technical feature of disposing the two handrails 33 on the stand platform 31 can provide convenience to the body composition data measurement for the measured person who needs an auxiliary or handrails to stand.

In addition, in the body composition analysis system 10 having image scanning function provided by the present invention, the technical feature of the two telescopic rods 331 and the two telescopic rods 331 being pivotably attached to the stand platform 31 enables the angles of the two handrails 33 adjustment according to the body shape of the measured person to maintain the posture of the measured person at the most appropriate measured condition and provide the measured person support so that the measured person is not liable to swing or change posture, that can make the measurement results have repeatability and improve the accuracy thereof.

In the first preferred embodiment, the two first electrode sets 313 and the two second electrode sets 335 are eight-electrode mode. The two first electrode sets 313 and the two second electrode sets 335 have respective current sensing electrode and current emitting electrode.

In the first preferred embodiment, the image capturing device 20 includes an image capturing lens 21 and a Y-axial stand 23. The image capturing lens 21 is pivotably attached to the Y-axial stand 23 and movable along Y-axis, thereby increasing the image capturing angle of the image capturing lens 21. In practical implementation, in the condition that the image capturing angle of the image capturing lens 21 is large enough, the image capturing device 20 may be fixed, so the Y-axial stand 23 is not an essential condition for attaining the present invention.

In the first preferred embodiment, the stand platform 31 is further provided with a rotary plate 315. The rotary plate 315 may be located at the top surface of the stand platform 31 for the measured person to stand thereon. In this way, under the condition that the measured person is maintained at the correct measured posture, the measured person can be turn by the rotary plate 315 for the image capturing lens 21 to shoot from different angles. In practical implementation, the rotary plate 315 may be located at the bottom of the stand platform 31, so that under the condition that the measured person is maintained at the correct measured posture, the measured person can be also turn by the rotary plate 315 for the image capturing lens 21 to shoot from different angles. Therefore, the rotary plate 315 is unlimited to be implemented as the type disclosed in this preferred embodiment.

In the first preferred embodiment, the image capturing lens 21 is a depth camera, which can use a plurality of images of the whole or partial body obtained from different angles or different distances and use three-dimensional reconstruction technology to obtain the surface shape of the body of the measured person by calculation, but it may be implemented by a normal camera or a plurality of normal cameras in practical implementation. Therefore, the image capturing device 20 is unlimited to be implemented as the type disclosed in this preferred embodiment.

In the first preferred embodiment, the calculating unit 35 further includes a determining logic 355 to determine whether the image data obtained by the image capturing device 20 is correct or not by data in the body shape module database 351. In practical implementation, if determining whether the posture of the measured person is correct or not is not required, the disposal of the determining logic 355 can be omitted, so the determining logic 355 is not an essential condition for attaining the present invention.

In the first preferred embodiment, the aforementioned another end of each of the telescopic rods 331 is pivotably attached to each of the handles 333, so that each of the handles 333 is adjustable according to the body shape of the measured person to the best hold condition for the measured person for the measurement. However, if the adjustment provided by the two telescopic rods 331 being pivotably attached to the stand platform 31 has enabled the standard posture for the measurement, the technical feature of the aforementioned another end of each of the telescopic rods 331 being pivotably attached to each of the handles 333 can be omitted. Therefore, the technical feature of the aforementioned another end of each of the telescopic rods 331 being pivotably attached to each of the handles 333 is not an essential condition for attaining the present invention.

In the first preferred embodiment, the stand platform 31 is further provided with a weight sensor 317. The weight sensor 317 is electrically connected with the calculating unit 35, so that the weight of the measured person can be directly measured by the body composition analyzer 30 and thereby the weight data can be provided to the calculational logic 353 by transmission for increasing the accuracy of the body composition data measured by the present invention. In practical implementation, if the manner of the measured person self-entering the weight thereof is adopted, the disposal of the weight sensor 317 can be omitted, so the weight sensor 317 is not an essential condition for attaining the present invention.

In the first preferred embodiment, the control panel 40 is adapted for the entering of the information of the measured person, such as age, sex, weight, height and race, and transmitting it to the calculational logic 353, that can also increase the accuracy of the body composition data measured by the present invention. In practical implementation, if the height is not entered, it can be calculated by the reconstructed three-dimensional model.

In the first preferred embodiment, the two first electrode sets 313 and the two second electrode sets 335 use alternating current of different frequencies to measure resistance and reactance of each limb segment of the measured person. The image data obtained by the image capturing device 20 includes circumferences, cross-sectional areas and lengths of different limb segments.

Wherein, the relations between the resistance (R) and the reactance (Xc) and between the impedance and the resistance and reactance corresponding to each limb segment can be respectively represented as Z=(R²+Xc²)^{1/2} and ArcTan(Xc/R)=PhA, wherein ArcTan and PhA are arctangent function and phase angle respectively. The larger the phase angle, the better the health of the measured person. For different limb segments, the larger the phase angle, the better the 'quality' of the associated body composition.

Wherein, the measurement of the body composing components is performed to each limb segment by its circumference, cross-sectional area, length and the resistance and reactance obtained by measurements corresponding to different frequencies, but not only the impedance of single frequency. That makes the measurement for different body composing components relatively more accurate. In this preferred embodiment, the combination of very low current and different frequencies (1KHz-3000KHz) is applied to the resistance and reactance measurement for the whole body and the left, right, upper and lower limb segments of the measured person. Not only the resistance and reactance of different frequencies can be obtained, they can be also combined and calculated to obtain the impedance. For the body composition measurement, there are relatively more corresponding variables, so that the body composition measurement is relatively more accurate.

By the alternating current of different frequencies, the corresponding values obtained by the measurements of the resistance and reactance of the whole body and each limb segment of the measured person further enable the measurement of the body water, intracellular fluid and extracellular fluid of the whole body and each limb segment of the measured person. Externally, it can be the outputted measurement results of the body composition. For the device itself, the measurement results of the resistance and reactance corresponding to the different frequencies are applicable to correct the body water distribution of the standing measured person, reducing the variability of the measurement results using BIA.

In this preferred embodiment, by the corresponding values obtained by the measurements of the resistance and reactance of the whole body and each limb segment of the measured person by the alternating current of different frequencies, in coordination with the height of the measured person or the length of each limb segment obtained after the image capturing device 20 obtains the image data, bioelectrical impedance vector analysis for the whole body or each limb segment of the measured person is performed. The impedance vector analysis is performed by corresponding the height of the whole body of the measured person to the resistance and reactance corresponding to the whole body, or corresponding the length of each limb segment. Under the precondition without the need to apply empirical equation or regression equation, R/H and Xc/H can be applied to perform assessment and analysis of the body composition of the whole body or each limb segment in hydration status and muscle mass.

Except for the aforementioned bioelectrical impedance vector analysis, in this preferred embodiment, special bioelectrical impedance vector analysis for the whole body or each limb segment of the measured person may be also performed. By corresponding the height of the whole body to the resistance and reactance corresponding to the whole body or corresponding the length of each limb segment, the special bioelectrical impedance vector analysis is performed. Under the precondition without the need to apply empirical equation or regression equation, Rsp and Xcsp are applied to perform assessment and analysis of the body composition of the whole body or each limb segment in hydration status and muscle mass, wherein Rsp and Xcsp are the whole body or limb segment multiplied by the corresponding correction factor (cross-section/length).

The structure of the first preferred embodiment of the present invention is described above. The usage condition of the first preferred embodiment of the present invention is described in the following.

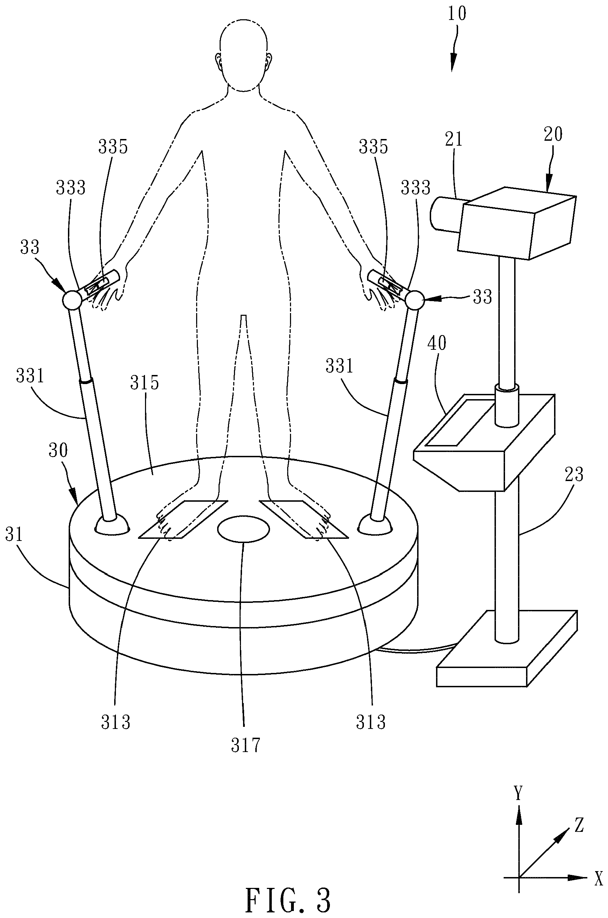

As shown in FIGS. 2 and 3, at first, the information of the measured person, such as age, sex, height and race, is entered through the control panel 40.

Then, the measured person stands onto the stand platform 31. The pressure formed by the weight of the measured person makes the left and right soles of the measured person in contact with the two first electrode sets 313 respectively, and then the weight data of the measured person is obtained by the weight sensor 317 and transmitted to the calculating unit 35.

After that, the both hands of the measured person contact the two second electrode sets 335 respectively by holding the two handles 333. At the same time, by the technical features that the two telescopic rods 331 can be lengthened and shortened, the junctures of the two telescopic rods 331 and the stand platform 31 are pivotable, and the two handles 333 are pivotable, the posture of the measured person can be adjusted to the most appropriate measured condition and the measured person is provided with support so that the measured person is not liable to swing or change posture.

After that, the rotary plate 315 starts to rotate, and the image capturing lens 21 starts to capture the images of the measured person and perform the human body three-dimensional model reconstruction by the images of different angles of the measured person obtained by the image capturing. After the reconstruction is accomplished, it is transmitted to the calculating unit 35. The determining logic 355 compares the model data in the body shape module database 351 with the image data obtained by the image capturing lens 21 to determine the correctness. If it is determined incorrect by the comparison, it means the measured posture of the measured person is incorrect, and the calculating unit 35 will not perform the calculation, which means the present invention will not perform the measurement. If the result of the determining logic 355 determining the image data obtained by the image capturing lens 21 by the comparison with the model data in the body shape module database 351 is it has a little error, the calculating unit 35 uses the most similar model data in the body shape module database 351 to correct the image data obtained by the image capturing lens 21, and obtains the body composition data by calculation, thereby increasing the accuracy. If the result of the determining logic 355 determining the image data obtained by the image capturing lens 21 by the comparison with the model data in the body shape module database 351 is it is correct, the calculating unit 35 needs not to correct, but directly calculates to obtain the body composition data.

After that, if the determining logic 355 determines the posture of the measured person is correct, by electrode plate switching mechanism of the two first electrode sets 313 and the two second electrode sets 335, alternating current of different combinations and different frequencies is applied to function with the current emitting and sensing electrodes. The resistance and reactance of different frequencies at different limb segments can be measured, and after the image data is compared with the body shape module database 351, the calculational logic 353 calculates to obtain the body composition data.

As a result, in the body composition analysis system 10 having image scanning function provided by the present invention, the technical feature of disposing the two handrails 33 on the stand platform 31 can provide convenience to the body composition data measurement for the measured person who needs an auxiliary or handrails to stand.

Besides, in the body composition analysis system 10 having image scanning function provided by the present invention, the technical feature of the two telescopic rods 331 and the two telescopic rods 331 being pivotably attached to the stand platform 31 enables the adjustment according to the body shape of the measured person to maintain the posture of the measured person at the most appropriate measured condition and provide the measured person support so that the measured person is not liable to swing or change posture, that can improve the repeatability and accuracy of the measurement results.

A body composition analysis system 10' having image scanning function provided by a second preferred embodiment of the present invention is approximately similar to the above first preferred embodiment, but they have the difference described hereunder.

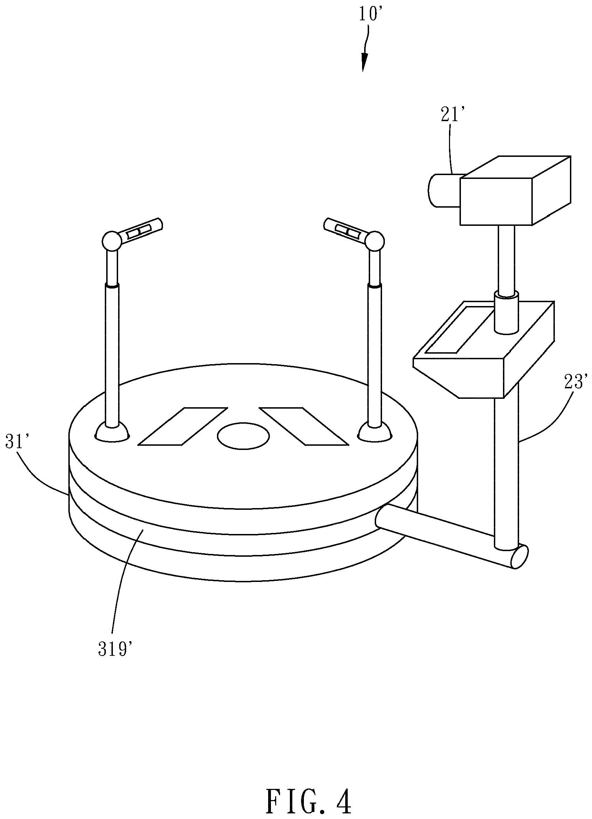

As shown in FIG. 4, this second preferred embodiment has no such rotary plate 315 in the first preferred embodiment. In this preferred embodiment, the stand platform 31' has a circular shape, and the stand platform 31' is provided along the outer periphery thereof with an annular guide rail 319'. The Y-axial stand 23' is disposed on the annular guide rail 319' and movable along the annular guide rail 319'.

In use, the image capturing lens 21' is moved in a manner of surrounding the stand platform 31', so that when the image capturing lens 21' captures the images of the measured person, the measured person will not be rotated and thereby swing or change posture. That can further improve the repeatability and accuracy of the measurement results of the present invention, especially for the measured person who is weak and even unable to stand.

Other structures and attained effects of this second preferred embodiment are the same with those of the first preferred embodiment, thereby not repeatedly described.

## Claims

1. A body composition analysis system (10, 10') having image scanning function, the body composition analysis system (10, 10') comprising
an image capturing device (20);
a body composition analyzer (30) distanced from the image capturing device (20) for a predetermined distance, the body composition analyzer (30) having a stand platform (31), two handrails (33) and a calculating unit (35), the stand platform (31) being provided with two first electrode sets (313), the two handrails (33) each having a telescopic rod (331) and a handle (333), an end of each of the telescopic rods (331) being pivotably attached to the stand platform (31), each of the handles (333) being disposed at another end of each of the telescopic rods (331), each of the handles (333) having a second electrode set (335), the calculating unit (35) being electrically connected with the image capturing device (20), the calculating unit (35) comprising a database (351), comprising data related to body shape stored therein, and a calculational logic (353), the calculating unit (35) receiving resistance and reactance measured by the two first electrode sets (313) and the two second electrode sets (335) and receiving image data obtained by the image capturing device (20), and after comparing the image data obtained by the image capturing device (20) with the data in the database (351), calculating by the calculational logic (353) to obtain a body composition data; and
a control panel (40) electrically connected with the body composition analyzer (30), the image capturing device (20) and the calculating unit (35) for showing the body composition data.

2. The body composition analysis system (10, 10') as claimed in claim 1, **characterized in that** the image capturing device (20) comprises an image capturing lens (21, 21') and a Y-axial stand (23); the image capturing lens (21, 21') is pivotably attached to the Y-axial stand (23) and movable along Y-axis.

3. The body composition analysis system (10, 10') as claimed in claim 2, **characterized in that** the stand platform (31) has a circular shape; the stand platform (31) is provided along an outer periphery thereof with an annular guide rail (319'); the Y-axial stand (23) is disposed on the annular guide rail (319') and movable along the annular guide rail (319').

4. The body composition analysis system (10, 10') as claimed in claim 1, **characterized in that** the stand platform (31) is further provided with a rotary plate (315).

5. The body composition analysis system (10, 10') as claimed in claim 1, **characterized in that** said another end of each of the telescopic rods (331) is pivotably attached to each of the handles (333).

6. The body composition analysis system (10, 10') as claimed in claim 1, **characterized in that** the stand platform (31) is further provided with a weight sensor (317); the weight sensor (317) is electrically connected with the calculating unit (35).

7. The body composition analysis system (10, 10') as claimed in claim 1, **characterized in that** the calculating unit (35) further comprises a determining logic (355) to determine whether the image data obtained by the image capturing device (20) is correct or not by data in the database (351).

8. The body composition analysis system (10, 10') as claimed in claim 1, **characterized in that** the two first electrode sets (313) and the two second electrode sets (335) use alternating current of different frequencies to measure resistance and reactance of each limb segment; the image data obtained by the image capturing device (20) comprises circumference, cross-sectional area and length of said each limb segment.

## Patentansprüche

1. System (10, 10') zur Analyse der Körperzusammensetzung mit Bildabtastfunktion, worin das System (10, 10') zur Analyse der Körperzusammensetzung umfasst:
eine Bildaufnahmevorrichtung (20);
einen Analysator (30) für die Körperzusammensetzung, der von der Bildaufnahmevorrichtung (20) um einen bestimmten Abstand entfernt ist, worin der Analysator (30) für die Körperzusammensetzung eine Standplattform (31), zwei Handläufe (33) und eine Recheneinheit (35) aufweist, worin die Standplattform (31) mit zwei ersten Elektrodensätzen (313) versehen ist, die beiden Handläufe (33) jeweils eine Teleskopstange (331) und einen Griff (333) aufweisen, worin ein Ende jeder der Teleskopstangen (331) schwenkbar an der Standplattform (31) befestigt ist, jeder der Griffe (333) an einem anderen Ende jeder der Teleskopstangen (331) angeordnet ist und jeder der Griffe (333) einen zweiten Elektrodensatz (335) aufweist, die Recheneinheit (35) mit der Bildaufnahmevorrichtung (20) elektrisch verbunden ist, worin die Recheneinheit (35) eine Datenbank (351), die darin gespeicherte, auf die Körperform bezogene Daten umfasst, und eine Berechnungslogik (353) umfasst, die Recheneinheit (35) Widerstand und Reaktanz empfängt, die durch die zwei ersten Elektrodensätze (313) und die zwei zweiten Elektrodensätze (335) gemessen werden, und Bilddaten empfängt, die durch die Bildaufnahmevorrichtung (20) erhalten werden, und nach dem Vergleichen der Bilddaten, die durch die Bildaufnahmevorrichtung (20) erhalten werden, mit den Daten in der Datenbank (351) durch die Berechnungslogik (353) rechnet, sodass Daten über die Körperzusammensetzung erhalten werden; und
ein Bedienfeld (40), das mit dem Körperzusammensetzungsanalysator (30), der Bildaufnahmevorrichtung (20) und der Recheneinheit (35) elektrisch verbunden ist, um die Daten der Körperzusammensetzung anzuzeigen.

2. System (10, 10') zur Analyse der Körperzusammensetzung nach Anspruch 1, **dadurch gekennzeichnet, dass** die Bildaufnahmevorrichtung (20) eine Bildaufnahmelinse (21, 21') und ein Y-axiales Stativ (23) umfasst, worin die Bildaufnahmelinse (21, 21') an dem Y-axialen Stativ (23) schwenkbar angebracht und entlang der Y-Achse beweglich ist.

3. System (10, 10') zur Analyse der Körperzusammensetzung nach Anspruch 2, **dadurch gekennzeichnet, dass** die Standplattform (31) eine kreisförmige Form hat; die Standplattform (31) entlang ihres Außenumfangs mit einer ringförmigen Führungsschiene (319') versehen ist; das Y-axiale Stativ (23) auf der ringförmigen Führungsschiene (319') angeordnet und entlang der ringförmigen Führungsschiene (319') bewegbar ist.

4. System zur Analyse der Körperzusammensetzung (10, 10') nach Anspruch 1, **dadurch gekennzeichnet, dass** die Standplattform (31) ferner mit einem Drehteller (315) versehen ist.

5. System zur Analyse der Körperzusammensetzung (10, 10') nach Anspruch 1, **dadurch gekennzeichnet, dass** das andere Ende jeder der Teleskopstangen (331) schwenkbar an jedem der Griffe (333) befestigt ist.

6. System zur Analyse der Körperzusammensetzung (10, 10') nach Anspruch 1, **dadurch gekennzeichnet, dass** die Standplattform (31) ferner mit einem Gewichtssensor (317) versehen ist; und der Gewichtssensor (317) mit der Recheneinheit (35) elektrisch verbunden ist.

7. System zur Analyse der Körperzusammensetzung (10, 10') nach Anspruch 1, **dadurch gekennzeichnet, dass** die Recheneinheit (35) ferner eine Bestimmungslogik (355) umfasst, um anhand von Daten in der Datenbank (351) zu bestimmen, ob die von der Bildaufnahmevorrichtung (20) erhaltenen Bilddaten korrekt sind oder nicht.

8. System (10, 10') zur Analyse der Körperzusammensetzung nach Anspruch 1, **dadurch gekennzeichnet, dass** die beiden ersten Elektrodensätze (313) und die beiden zweiten Elektrodensätze (335) Wechselstrom mit unterschiedlichen Frequenzen verwenden, um den Widerstand und die Reaktanz jedes Gliedmaßensegments zu messen; und dadurch dass die von der Bildaufnahmevorrichtung (20) erhaltenen Bilddaten den Umfang, die Querschnittsfläche und die Länge jedes Gliedmaßensegments umfassen.

## Revendications

1. Système d'analyse de la composition corporelle (10, 10') ayant une fonction de balayage d'image, le système d'analyse de la composition corporelle (10, 10') comprenant:
un dispositif de capture d'images (20);
un analyseur de composition corporelle (30) éloigné du dispositif de capture d'images (20) d'une distance prédéterminée, l'analyseur de composition corporelle (30) ayant une plate-forme (31), deux mains courantes (33) et une unité de calcul (35), la plate-forme (31) étant pourvue de deux premiers ensembles d'électrodes (313), les deux mains courantes (33) comportent chacune une tige télescopique (331) et une poignée (333), une extrémité de chacune des tiges télescopiques (331) étant fixée de manière pivotante à la plate-forme (31), chacune des poignées (333) étant disposée à une autre extrémité de chacune des tiges télescopiques (331), chacune des poignées (333) comportant un second jeu d'électrodes (335), l'unité de calcul (35) est connectée électriquement au dispositif de capture d'images (20), l'unité de calcul (35) comprend une base de données (351), comprenant des données relatives à la forme du corps qui y sont stockées, et une logique de calcul (353), l'unité de calcul (35) reçoit la résistance et la réactance mesurées par les deux premiers ensembles d'électrodes (313) et les deux seconds ensembles d'électrodes (335) et reçoit les données d'image obtenues par le dispositif de capture d'images (20), et après avoir comparé les données d'image obtenues par le dispositif de capture d'images (20) avec les données de la base de données (351), calcule par la logique de calcul (353) pour obtenir des données sur la composition corporelle; et
un panneau de commande (40) connecté électriquement à l'analyseur de composition corporelle (30), au dispositif de capture d'images (20) et à l'unité de calcul (35) pour afficher les données relatives à la composition corporelle.

2. Système d'analyse de la composition corporelle (10, 10') selon la revendication 1, **caractérisé en ce que** le dispositif de capture d'images (20) comprend un objectif de capture d'images (21, 21') et un support d'axe Y (23); l'objectif de capture d'images (21, 21') est fixé de manière pivotante au support d'axe Y (23) et peut être déplacé le long de l'axe Y.

3. Système d'analyse de la composition corporelle (10, 10') selon la revendication 2, **caractérisé en ce que** la plate-forme de support (31) a une forme circulaire; la plate-forme de support (31) est pourvue sur sa périphérie extérieure d'un rail de guidage annulaire (319'); le support de l'axe Y (23) est disposé sur le rail de guidage annulaire (319') et peut être déplacé le long du rail de guidage annulaire (319').

4. Système d'analyse de la composition corporelle (10, 10') selon la revendication 1, **caractérisé en ce que** la plate-forme (31) est en outre pourvue d'un plateau rotatif (315).

5. Système d'analyse de la composition corporelle (10, 10') selon la revendication 1, **caractérisé en ce que** l'autre extrémité de chacune des tiges télescopiques (331) est fixée de manière pivotante à chacune des poignées (333).

6. Système d'analyse de la composition corporelle (10, 10') selon la revendication 1, **caractérisé en ce que** la plate-forme (31) est en outre équipée d'un capteur de poids (317); le capteur de poids (317) est relié électriquement à l'unité de calcul (35).

7. Système d'analyse de la composition corporelle (10, 10') selon la revendication 1, **caractérisé en ce que** l'unité de calcul (35) comprend en outre une logique de détermination (355) pour déterminer si les données d'image obtenues par le dispositif de capture d'images (20) sont correctes ou non en fonction des données de la base de données (351).

8. Système d'analyse de la composition corporelle (10, 10') selon la revendication 1, **caractérisé en ce que** les deux premiers ensembles d'électrodes (313) et les deux seconds ensembles d'électrodes (335) utilisent un courant alternatif de fréquences différentes pour mesurer la résistance et la réactance de chaque segment de membre; les données d'image obtenues par le dispositif de capture d'images (20) comprennent la circonférence, la surface de section transversale et la longueur de chaque segment de membre.
